# EUROPEAN PATENT SPECIFICATION

(11) **EP 1 366 714 B1**
(45) Date of publication and mention of the grant of the patent: **15.02.2017**
(21) Application number: 03015376.1
(22) Date of filing: 24.10.1997
(51) Int. Cl.: A61B 6/04, A61N 5/10

(54) **Accessory clamping system for couch top**
Klemmzusatzsystem für eine Patientenliege
Système servant a serrer un accessoire sur le dessus d'une table de soins

(30) Priority: 25.10.1996 US 738429
(43) Date of publication of application: 03.12.2003
(62) Divisional of application: 97913771.8
(73) Proprietor: Varian Medical Systems, Inc., Palo Alto, CA 94304 (US)
(72) Inventor: Oliver, Edward A., Folsom, CA 95630 (US); Haugher, Todd M., Orange City, IA 51041 (US)
(74) Representative: Foster, Mark Charles

(56) References cited:
- FR-A- 2 523 433
- US-A- 2 301 804
- US-A- 3 405 931
- US-A- 3 700 229
- US-A- 4 394 800
- US-A- 4 852 841
- US-A- 5 152 486
- US-A- 5 276 927

## Description

### BACKGROUND OF THE INVENTION

This invention relates to a system for clamping to a couch top an accessory such as a patient immobilization device for head and neck treatments or stereotactic radiotherapy.

Current trends in general purpose radiotherapy are directed towards more clinically efficient setups and treatments. As a result, devices for locating patients require precise and repeatable repositioning of six (three translational and three rotational) degrees of freedom with quick and easy attachment. In addition, there exists a need for flexibility within the locating system to accommodate a broad range of body shapes and treatment sites.

A precise and repeatable indexing system that is quick and easy to attach is currently not available. Attachment schemes tend to be incorporated either with minor modifications or non-repeatable clamping arrangements, and not directly integrated into the couch top design.

Some more advanced prior art systems clamp to accessory rails which are found on most radiotherapy couches. Accessory rails are generally fixed lengthwise along the side of the couch top. The rail attachment itself will generally secure all six degrees of freedom, but not repeatably. Manual adjustment is required to re-establish at least one translational and one rotational degrees of freedom once the device is removed.

Some devices possess an additional interface between such an accessory rail clamping system and the actual accessory. Pins protruding from either the rail clamping system or the accessory mate with clearance holes. This provides for one translational and two rotational degrees of freedom precisely secured and two translational and one rotational degrees of freedom loosely secured. Although quick and easy to attach, this is not a precise and repeatable locating system as a whole or even at the subsystem level.

Locating systems that actually attach to the treatment surface are essentially custom jobs developed by individual hospital personnel. Pins protruding from either the couch top surface or the accessory mate with clearance holes. This also provides for one translational and two rotational degrees of freedom precisely secured and two translational and one rotational degrees of freedom loosely secured. Neither is this a precise and repeatable locating system since, by necessity, clearance exists between the hole and the pin.

Capturing two treatment surface edges is another means of locating. Flanges attached to the accessory protrude below the surface of the couch securing one translational and two rotational degrees of freedom precisely and one translational and one rotational degrees of freedom loosely. This leaves one last degree of freedom left unsecured.

Stereotactic head frames, although not considered for general purpose radiotherapy, do lock down six degrees of freedom, but they are not quick and easy to attach, being bolted in most cases. They do not secure to the treatment surface because a higher precision is required than in general purpose radiotherapy. Attachment is generally to the frame. These devices typically require a separate manual adjustment for precision fine tuning of position by using an external reference such as a laser.

FR-A-2 523 433 discloses a patient support that is rotatably coupled to, and supported by, a frame.

US-A-4 852 841 discloses a device for clamping to an edge of a table. The device has a top and bottom jaws for contacting top and bottom surfaces, respectively, of the table.

US-A-4 394 800 discloses a guide clamp for clamping to a workpiece. The guide clamp includes a clamping jaw that is used to clamp against opposite sides of a workpiece.

US-A-3 405 931 discloses a hammock configured for being mounted to an operating table. The hammock includes a clamp at a bottom, which is used to clamp agains opposite sides of the operating table.

US-A-5 276 927 discloses a patient support table having side rails on opposite sides o the patient support table, and a head support slidably mounted to the side rails.

US-A-5 152 486 discloses a surgical table having rails on opposite sides of the surgica table, and a surgical assistance device slidably mounted to the side rails.

US-A-3 700 229 discloses an x-ray table that includes fastening strips secured to the sides of the table via slidable pieces. The slidable pieces have a T-shaped configuration, which prevents disengagement of the fastening strips from the table.

US-A-2 301 804 discloses a bag holding apparatus configured to hold bags in position during the operation of filling or discharging material into the bags.

### SUMMARY OF THE INVENTION

It is therefore an object of this invention to provide an improved clamping systen capable of quickly and easily clamping an accessory such as a patient immobilization device to a couch top for the patient with precision and repeatability.

According to one aspect of the invention, there is provided an accessory clamping system as defined in claim 1.

According to another aspect of the invention, there is provided an accessory clamping system as defined in claim 10.

### BRIEF DESCRIPTION OF THE DRAWINGS

The accompanying drawings, which are incorporated in and form a part of this specification, illustrate an embodiment of the invention and, together with the description, serve to explain the principles of the invention. In the drawings:
Fig. 1 is an diagonal view of an accessory clamping system embodying this invention when a bar, which may be a part of an accessory to be clamped, is clamped to a couch top;
Fig. 2 is an diagonal view of a portion of the accessory clamping system of Fig. 1 when the bar is about to be clamped to the couch top; and
Fig. 3 is an enlarged plan view of the eccentrically mounted disk and the lever of the clamping system of Figs. 1 and 2.

### DETAILED DESCRIPTION OF THE INVENTION

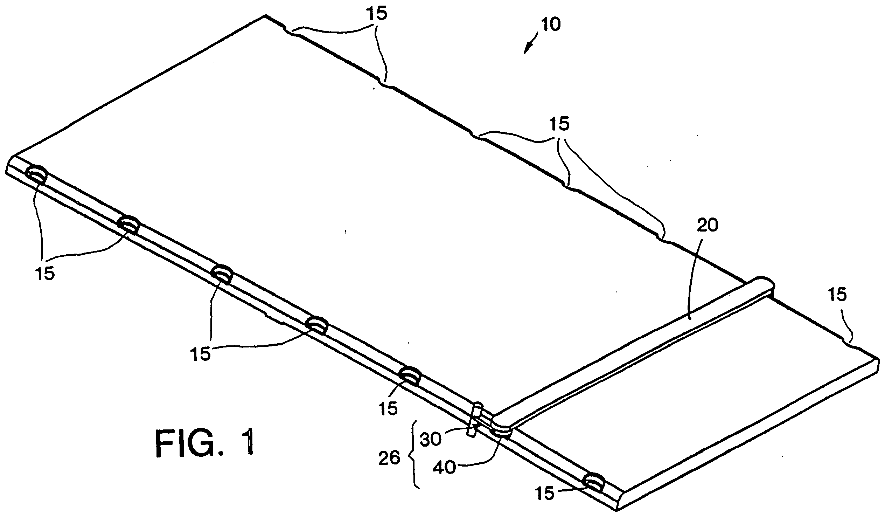

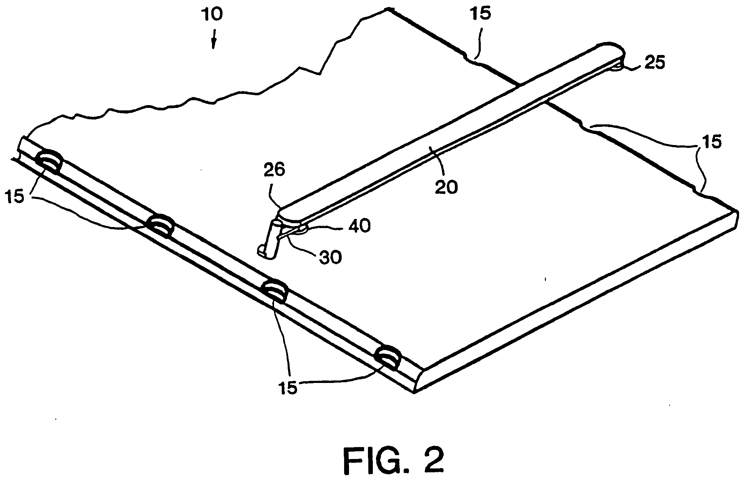

In Figs. 1 and 2, numeral 10 indicates a generally rectangular panel on a couch top, to which an accessory such as a patient immobilization device is adapted to be secured. The panel 10 is provided with pairs of indexing indentations 15 on mutually opposite side edges, each pair having one of the indentations on one of the side edges and the other on the other of the side edges such that each pair of the indexing indentations 15 is transversely across the panel surface opposite to each other. According to a preferred embodiment of the invention, these indentations 15 are crescent-shaped and convex towards the other of the pair.

Numeral 20 indicates a bar which is to be secured onto the panel 10 by making use of these indexing indentations 15. It is approximately of the same length as the width of the rectangular panel 10, that is, its length is about the same as the separation between each pair of the indexing indentations 15 and may be regarded as a part of an accessory such as a patient immobilization device or a frame to which such an accessory is adapted to be securely attached.

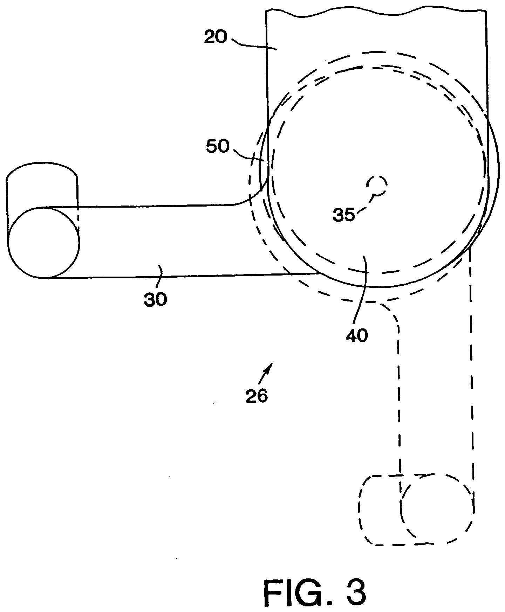

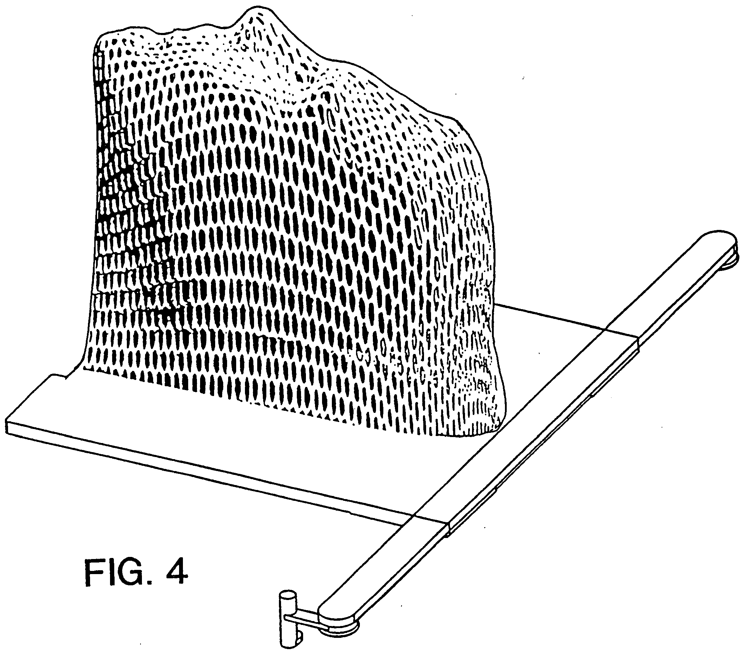

Attached to the lower surface of the bar 20 at one of its ends is a circular disk-shaped member 25, herein referred to as the fixed disk, adapted to fit inside any of these indexing indentations 15. A lever 30 is provided at the other end of the bar 20, attached to a vertical shaft 35 which is rotatably supported by the bar 20 and to which a circular disk 40 is eccentrically mounted, as shown in Fig. 3. Thus, this eccentrically mounted disk 40 is adapted to rotate with and around the shaft 35 such that, when the lever 30 extends perpendicularly to the direction of extension of the bar 20 as shown by solid line in Fig. 3, the center of the circular disk 40 is on the side of the shaft 35 towards the other end of the bar 20.

Quick and easy attachment of the bar 20 to the panel 10 is accomplished by first rotating the lever 30 by 90 degrees to the direction away from the other end of the bar 20, as shown by dotted lines in Fig. 3, thereby also moving the eccentrically mounted disk 40 away from the other end of the bar 20, that is, increasing the distance between the two disks 25 and 40 on the bar 20 by a small distance determined by the eccentricity of the mounting of the rotatable disk 40. With the lever 30 thus oriented, the bar 20 is positioned as shown in Fig. 2 such that the fixed disk 25 and the eccentrically mounted disk 40 are each fit inside a corresponding one of a selected pair of the indexing indentations 15. Thereafter, the lever 30 is rotated such that the eccentrically mounted disk 40 will move towards the other end of the bar 12, thereby reducing the distance of separation between the fixed disk 25 and the eccentrically mounted rotatable disk 40. The separation between the pairs of indentations 15, the distance between the fixed disk 25 and the dimensions of the rotatable disk 40 including the eccentricity of its mounting to the shaft 35 are so determined that this rotation of the lever 30 causes the bar 20 to be clamped securely onto the panel 10 between the selected pair of indentations 15. In other words, the attachment is effected by a simple rotation by 90 degrees of the lever 30 from an unlocked position to a locked position. When locked, a clamp force is generated which effectively secures all three translational and three rotational degrees of freedom. Since the crescent-shaped indentations 15 are fixed, positioning is definitively precise and repeatable.

The invention has been described above with reference to only one example but the illustrated example is not intended to limit the scope of this invention. Many modifications and variations are possible within the scope of the invention. For example, the fixed disk 35 may be replaced by a member having a different shape, and the indentations 15 need not necessarily be crescent-shaped. Indentations of any shape capable of properly accepting and engaging such a fixed member to lock the bar can serve the purpose of this invention. The lever 30 may be replaced by any means for causing the eccentrically mounted disk 40 to be rotated around the shaft 35, including a cap adapted to start slipping after a certain maximum torque has been reached. As shown in Fig. 3, an O-ring 50 may be used around the eccentrically mounted disk 40, as well as around the fixed disk 25 at the interface with the inner walls of the indentations 15 so as to distribute clamp force, to reduce wear, to accommodate variations in spacing with the arcuate inner walls of the indentations 15 and to keep the eccentrically mounted disk 40 from loosening by increasing sliding friction.

The language in the specification, as well as in the claims, is intended to be interpreted broadly. The expression "indentations", for example, is intended to include configurations which may look more like a hole or a groove, as long as they have inner walls facing away from each other such that the fixed disk 25, or a member of any other shape which can reasonably substitute for it, and the eccentrically mounted disk 40 at opposite ends of the bar 20, as described above, can be received therein and cause the bar 20 to be locked to the panel 10.

It is also to be reminded that the bar 20, as used above in the description, may be only a portion of the accessory to be clamped to the panel 10 or a mere representation of any target device which is intended to be clamped to the panel 10 and hence need not have all regular attributes of what one generally calls "a bar" such as being elongated or defining a direction of extension.

## Claims

1. An accessory clamping system, comprising:
a patient support panel (10) configured for allowing a target device (20) to be coupled thereto, the patient support panel (10) supporting the target device (20) after the target device (20) is coupled thereto, the patient support panel (10) having a length along a longitudinal side of the patient support panel (10), and a pair of indentations that include a first indentation (15) and a second indentation (15), the target device (20) having a first member (40), a second member (25), and a rotation mechanism (30);
**characterized in that**:
each of the first and second indentations has inner walls facing away from each other;
the first member (40) comprises a first circular disk-shaped member that is eccentrically mounted;
the second member (25) comprises a second circular disk-shaped member;
the rotation mechanism (30) is configured to rotate the first circular disk-shaped member; and
the first and second circular disk-shaped members are configured to engage against the respective first and second indentations (15).

2. The accessory clamping system of claim 1, wherein the patient support panel (10) is a part of a couch top.

3. The accessory clamping system of claim 1 or 2, wherein one or both of the first and second indentations (15) comprises a hole or a groove.

4. The accessory clamping system of any one of claims 1 to 3, wherein each of the first and second indentations (15) has a curved surface.

5. The accessory clamping system of any one of claims 1 to 4, wherein the patient support panel (10) further comprises one or more additional pair(s) of indentations (15) to which the first and second members (25) can be engaged.

6. The accessory clamping system of any one of claims 1 to 5, wherein the first and second indentations (15) are located on opposite edges of the patient support panel (10).

7. The accessory clamping system of any one of claims 1 to 6, further comprising the target device (20).

8. The accessory clamping system of any one of claims 1 to 7, wherein the first member (25) is configured to be kept from loosening from the first indentation (15) by increasing sliding friction.

9. The accessory clamping system of any one of claims 7 to 8, wherein the patient support panel (10) includes additional pair(s) of indentations (15) to which the first member (25) can be engaged.

10. An accessory clamping system, comprising:
a target device (20) configured to be coupled to the patient support panel (10) as defined in claim 1, the target device (20) supported by the patient support panel (10) after the target device (20) is coupled to the patient support panel (10), the patient support panel (10) having a length along a longitudinal side, and a pair of indentations that include a first indentation and a second indentation (15), the target device (20) having a first end, a second end, a first member (40) coupled to the first end, and a second member (25) coupled to the second end;
**characterized in that**:
each of the first and second indentations (15) has inner walls facing away from each other;
the first member (40) comprises a first circular disk-shaped member that is eccentrically mounted;
the second member (25) comprises a second circular disk-shaped member;
the target device (20) further comprises a rotation mechanism (30) configured to rotate the first circular disk-shaped member; and
the first and second circular disk-shaped members are configured to engage against the respective first and second indentations (15) of the patient support panel.

11. The accessory clamping system of claim 10, wherein the target device (20) comprises at least a portion of an accessory.

12. The accessory clamping system of claim 10, wherein the target device (20) comprises a structure to which an accessory can be attached.

13. The accessory clamping system of claim 11 or 12, wherein the accessory is a patient immobilization device.

14. The accessory clamping system of any one of claims 10 to 13, wherein the target device (20) has a dimension that is approximately the same as a width of the patient support panel (10).

15. The accessory clamping system of any one of claims 10 to 14, wherein the first and second members (25) are separated by a distance which is approximately the same as a separation between the first and second indentations (15).

16. The accessory clamping system of any one of claims 10 to 15, wherein each of the first and second indentations (15) has a curved surface.

17. The accessory clamping system of any one of claims 10 to 16, wherein the first member (25) is configured to be kept from loosening from the first indentation (15) by increasing sliding friction.

18. The accessory clamping system of claim 10, wherein the first circular disk-shaped member is eccentrically mounted to a shaft (35) that is rotatably supported by the target device (20).

19. The accessory clamping system of claim 18, wherein the rotation mechanism is configured for rotating the first circular disk-shaped member around the shaft (35) to thereby secure the target device (20) to the panel (10).

20. The accessory clamping system of any one of claims 10 to 19, further comprising the patient support panel (10).

21. The accessory clamping system of any one of claims 10 to 20, wherein the patient support panel (10) includes additional pair(s) of indentations (15) to which the first and second members (25) can be engaged.

## Patentansprüche

1. Klemmzusatzsystem, umfassend:
eine Patiententragefläche (10), die dazu konfiguriert ist, zuzulassen, dass eine Zielvorrichtung (20) daran gekoppelt wird, wobei die Patiententragefläche (10) die Zielvorrichtung (20) abstützt, wenn die Zielvorrichtung (20) daran gekoppelt wurde, wobei die Patiententragefläche (10) eine Länge entlang einer Längsseite der Patiententragefläche (10) und ein Paar Vertiefungen aufweist, das eine erste Vertiefung (15) und eine zweite Vertiefung (15) beinhaltet, wobei die Zielvorrichtung (20) ein erstes Element (40), ein zweites Element (25) und einen Drehmechanismus (30) aufweist;
**dadurch gekennzeichnet, dass**:
die erste und zweite Vertiefung jeweils Innenwände aufweisen, die voneinander fort gewandt sind;
das erste Element (40) ein erstes kreisförmiges scheibenförmiges Element umfasst, das exzentrisch angebracht ist;
das zweite Element (25) ein zweites kreisförmiges scheibenförmiges Element umfasst;
der Drehmechanismus (30) dazu konfiguriert ist, das erste kreisförmige scheibenförmige Element zu drehen; und
das erste und zweite kreisförmige scheibenförmige Element dazu konfiguriert sind, an der jeweiligen ersten und zweiten Vertiefung (15) in Eingriff zu treten.

2. Klemmzusatzsystem nach Anspruch 1, wobei die Patiententragefläche (10) Teil einer Liegenoberseite ist.

3. Klemmzusatzsystem nach Anspruch 1 oder 2, wobei eine oder beide von der ersten und zweiten Vertiefung (15) ein Loch oder eine Nut umfassen.

4. Klemmzusatzsystem nach einem der Ansprüche 1 bis 3, wobei die erste und zweite Vertiefung (15) jeweils eine gekrümmte Fläche aufweisen.

5. Klemmzusatzsystem nach einem der Ansprüche 1 bis 4, wobei die Patiententragefläche (10) ferner ein oder mehrere weitere Paar(e) Vertiefungen (15) umfasst, in die das erste und zweite Element (25) eingreifen können.

6. Klemmzusatzsystem nach einem der Ansprüche 1 bis 5, wobei die erste und zweite Vertiefung (15) an gegenüberliegenden Kanten der Patiententragefläche (10) angeordnet sind.

7. Klemmzusatzsystem nach einem der Ansprüche 1 bis 6, ferner umfassend die Zielvorrichtung (20).

8. Klemmzusatzsystem nach einem der Ansprüche 1 bis 7, wobei das erste Element (25) dazu konfiguriert ist, an einem Lösen aus der ersten Vertiefung (15) gehindert zu werden, indem der Reibungswiderstand erhöht wird.

9. Klemmzusatzsystem nach einem der Ansprüche 7 bis 8, wobei die Patiententragefläche (10) weitere Paar(e) Vertiefungen (15) aufweist, in die das erste Element (25) eingreifen kann.

10. Klemmzusatzsystem, umfassend:
eine Zielvorrichtung (20), die dazu konfiguriert ist, an die Patiententragefläche (10) nach Anspruch 1 gekoppelt zu werden,
wobei die Zielvorrichtung (20) von der Patiententragefläche (10) abgestützt wird, wenn die Zielvorrichtung (20) an die Patiententragefläche (10) gekoppelt wurde, wobei die Patiententragefläche (10) eine Länge entlang einer Längsseite und ein Paar Vertiefungen aufweist, das eine erste Vertiefung und eine zweite Vertiefung (15) beinhaltet, wobei die Zielvorrichtung (20) ein erstes Ende, ein zweites Ende, ein erstes Element (40), das an das erste Ende gekoppelt ist, und ein zweites Element (25) aufweist, das an das zweite Ende gekoppelt ist;
**dadurch gekennzeichnet, dass**:
die erste und zweite Vertiefung (15) jeweils Innenwände aufweisen, die voneinander fort gewandt sind;
das erste Element (40) ein erstes kreisförmiges scheibenförmiges Element umfasst, das exzentrisch angebracht ist;
das zweite Element (25) ein zweites kreisförmiges scheibenförmiges Element umfasst;
die Zielvorrichtung (20) ferner einen Drehmechanismus (30) umfasst, der dazu konfiguriert ist, das erste kreisförmige scheibenförmige Element zu drehen; und
das erste und zweite kreisförmige scheibenförmige Element dazu konfiguriert sind, an der jeweiligen ersten und zweiten Vertiefung (15) der Patiententragefläche in Eingriff zu treten.

11. Klemmzusatzsystem nach Anspruch 10, wobei die Zielvorrichtung (20) wenigstens einen Abschnitt eines Zusatzes umfasst.

12. Klemmzusatzsystem nach Anspruch 10, wobei die Zielvorrichtung (20) eine Struktur umfasst, an der ein Zusatz befestigt werden kann.

13. Klemmzusatzsystem nach Anspruch 11 oder 12, wobei der Zusatz eine Patientenimmobilisierungsvorrichtung ist.

14. Klemmzusatzsystem nach einem der Ansprüche 10 bis 13, wobei die Zielvorrichtung (20) eine Abmessung aufweist, die etwa die gleiche wie eine Breite der Patiententragefläche (10) ist.

15. Klemmzusatzsystem nach einem der Ansprüche 10 bis 14, wobei das erste und zweite Element (25) mit einem Abstand voneinander entfernt sind, der etwa der gleiche wie eine Entfernung zwischen der ersten und zweiten Vertiefung (15) ist.

16. Klemmzusatzsystem nach einem der Ansprüche 10 bis 15, wobei die erste und die zweite Vertiefung (15) jeweils eine gekrümmte Fläche aufweisen.

17. Klemmzusatzsystem nach einem der Ansprüche 10 bis 16, wobei das erste Element (25) dazu konfiguriert ist, an einem Lösen aus der ersten Vertiefung (15) gehindert zu werden, indem der Reibungswiderstand erhöht wird.

18. Klemmzusatzsystem nach Anspruch 10, wobei das erste kreisförmige scheibenförmige Element exzentrisch an einer Welle (35) angebracht ist, die von der Zielvorrichtung (20) drehbar abgestützt wird.

19. Klemmzusatzsystem nach Anspruch 18, wobei der Drehmechanismus dazu konfiguriert ist, das erste kreisförmige scheibenförmige Element um die Welle (35) zu drehen, um dadurch die Zielvorrichtung (20) an der Tragefläche (10) zu sichern.

20. Klemmzusatzsystem nach einem der Ansprüche 10 bis 19, ferner umfassend die Patiententragefläche (10).

21. Klemmzusatzsystem nach einem der Ansprüche 10 bis 20, wobei die Patiententragefläche (10) weitere Paar(e) Vertiefungen (15) aufweist, in die das erste Element (25) eingreifen kann.

## Revendications

1. Système servant à serrer un accessoire comprenant :
un plateau de soutien d'un patient (10) conçu pour permettre à un dispositif cible (20) de s'y coupler, leplateau de soutien (10) supportant le dispositif cible (20) après le couplage du dispositif cible (20) au plateau, leplateau de soutien (10) ayant une certaine longueur du côté longitudinal du plateau de soutien (10), et une paire de renfoncements comprenant un premier renfoncement (15) et un second renfoncement (15), le dispositif cible (20) comportant un premier élément (40), un second élément (25) et un mécanisme de rotation (30) ;
**caractérisé en ce que** :
chacun des premier et second renfoncements comporte des parois internes orientées en direction opposée l'une à l'autre ;
le premier élément (40) comprend un premier élément circulaire en forme de disque qui est monté de manière excentrique ;
le second élément (25) comprend un second élément circulaire en forme de disque ;
le mécanisme de rotation (30)
est conçu pour faire tourner le premier élément circulaire en forme de disque ; et
les premier et second éléments circulaires en forme de disque sont conçus pour venir en prise avec chacun des premier et second renfoncements (15), respectivement.

2. Système servant à serrer un accessoire selon la revendication 1, dans lequel leplateau de soutien du patient (10) fait partie du dessus d'une table de soins.

3. Système servant à serrer un accessoire selon la revendication 1 ou 2, dans lequel les premier et second renfoncements (15) comprennentl'un ou l'autre ou chacun un orifice ou une rainure.

4. Système servant à serrer un accessoire selon l'lune quelconque des revendications 1 à 3, dans lequel les premier et second renfoncements (15) comprennent chacun une surface incurvée.

5. Système servant à serrer un accessoireselon l'une quelconque des revendications 1 à 4, dans lequel leplateau de soutien du patient (10)comprend en outre une ou plusieurs paires additionnelles de renfoncements (15) dans lesquels les premier et second éléments (25) peuvent venir en prise.

6. Système servant à serrer un accessoireselon l'une quelconque des revendications 1 à 5, dans lequel les premier et second renfoncements (15) sont situés sur les bords opposés du plateau de soutien du patient (10).

7. Système servant à serrer un accessoire selon l'une quelconque des revendications 1 à 6, comprenant en outre le dispositif cible (20).

8. Système servant à serrer un accessoire selon l'une quelconque des revendications 1 à 7, dans lequel le premier élément (25) est conçu pour ne pas s'écarter du premier renfoncement (15) en augmentant le frottement par glissement.

9. Système servant à serrer un accessoire selon la revendication 7 ou 8, dans lequel leplateau de soutien du patient (10) comprend une ou plusieurs paires additionnelles de renfoncements (15) dans lesquels le premier élément (25) peut venir en prise.

10. Système servant à serrer un accessoire comprenant :
un dispositif cible (20) conçu pour se coupler au plateau de soutien du patient (10) selon la revendication 1,
le dispositif cible (20) supporté par le plateau de soutien (10) après le couplage du dispositif cible (20) au plateau de soutien (10), le plateau de soutien (10) ayant une certaine longueur ducôté longitudinal, et une paire de renfoncements comprenant un premier renfoncement et un second renfoncement (15), le dispositif cible (20) comportant une première extrémité, une seconde extrémité, un premier élément (40) couplé à la première extrémité, et un second élément (25) couplé à la seconde extrémité ;
**caractérisé en ce que** :
chacun des premier et second renfoncements (15) comporte des parois internes orientées en direction opposée l'une à l'autre ;
le premier élément (40) comprend un premier élément circulaire en forme de disque qui est monté de manière excentrique ;
le second élément (25) comprend un second élément circulaire en forme de disque ;
le dispositif cible (20) comprend en outre un mécanisme de rotation (30) conçu pour faire tourner le premier élément circulaire en forme de disque ; et
les premier et second éléments circulaires en forme de disque sont conçus pour venir en prise avec chacun des premier et second renfoncements (15), respectivement, du plateau de soutien du patient.

11. Système servant à serrer un accessoire selon la revendication 10, dans lequel le dispositif cible (20) comprend au moins une partie d'un accessoire.

12. Système servant à serrer un accessoire selon la revendication 10, dans lequel le dispositif cible (20) comprend une structure à laquelle un accessoire peut être fixé.

13. Système servant à serrer un accessoire selon la revendication 11 ou 12, dans lequel l'accessoire est un dispositif d'immobilisation du patient.

14. Système servant à serrer un accessoire selon l'une quelconque des revendications 10 à 13, dans lequel le dispositif cible (20) a une dimension qui est à peu près la même qu'une largeur du plateau de soutien du patient (10).

15. Système servant à serrer un accessoire selon l'une quelconque des revendications 10 à 14, dans lequel les premier et second éléments (25) sont séparés d'une distance qui est à peu près la même que la distance séparant les premier et second renfoncements (15).

16. Système servant à serrer un accessoire selon l'une quelconque des revendications 10 à 15, dans lequel les premier et second renfoncements (15) comprennent chacun une surface incurvée.

17. Système servant à serrer un accessoire selon l'une quelconque des revendications 10 à 16, dans lequel le premier élément (25) est conçu pour ne pas s'écarter du premier renfoncement (15) en augmentant le frottement par glissement.

18. Système servant à serrer un accessoire selon la revendication 10, dans lequel le premier élément circulaire en forme de disque est monté de manière excentrique sur un axe (35) qui repose sur le dispositif cible (20) en pivotant.

19. Système servant à serrer un accessoire selon la revendication 18, dans lequel le mécanisme de rotation est conçu pour faire tourner le premier élément circulaire en forme de disque autour de l'axe (35) de manière à fixer fermement le dispositif cible (20) au plateau (10).

20. Système servant à serrer un accessoire selon l'une quelconque des revendications 10 à 19, comprenant en outre leplateau de soutien du patient (10).

21. Système servant à serrer un accessoire selon l'une quelconque des revendications 10 à 20, dans lequel le plateau de soutien du patient (10) comprend une ou plusieurs paires additionnelles de renfoncements (15) dans lesquels les premier et second éléments (25) peuvent venir en prise.
